# FASCICULE DE BREVET EUROPEEN

(11) **EP 4 313 918 B1**
(45) Date de publication et mention de la délivrance du brevet: **18.12.2024**
(21) Numéro de dépôt: 22718184.9
(22) Date de dépôt: 25.03.2022
(51) Int. Cl.: C07C 2/04, C07C 11/107, C07C 11/02

(54) **PROCEDE D'OLIGOMERISATION COMPRENANT UNE ETAPE DE RECYCLAGE D'UN SOLVANT PREALABLEMENT REFROIDI**
OLIGOMERISIERUNGSVERFAHREN MIT EINEM SCHRITT ZUR RÜCKFÜHRUNG EINES VORGEKÜHLTEN LÖSUNGSMITTELS
OLIGOMERISATION PROCESS COMPRISING A STEP OF RECYCLING A PRE-COOLED SOLVENT

(30) Priorité: 30.03.2021 FR 2103228
(43) Date de publication de la demande: 07.02.2024
(73) Titulaire: IFP Energies nouvelles, 92500 Rueil-Malmaison (FR)
(72) Inventeur: BREUIL, Pierre-Alain, 92852 RUEIL-MALMAISON CEDEX (FR); COTTE, Olivier, 92852 RUEIL-MALMAISON CEDEX (FR)
(74) Mandataire: IFP Energies nouvelles
(86) Numéro de dépôt international: PCT/EP2022/057937
(87) Numéro de publication internationale: WO 2022/207495

(56) Documents cités:
- GB-A- 2 326 885
- US-A1- 2020 001 266

## Description

### Domaine technique

La présente invention concerne un procédé d'oligomérisation d'une charge oléfinique caractérisé en ce qu'une fraction solvant issue d'une étape aval de séparation est refroidie et recyclée. En particulier, la présente invention concerne un procédé d'oligomérisation d'une charge oléfinique gazeuse, de préférence d'éthylène gazeux, en alpha-oléfines linéaires telles que le but-1-ène, le hex-1-ène, ou l'oct-1-ène ou un mélange d'alpha-oléfines linéaires.

### Art antérieur

L'invention concerne le domaine de l'oligomérisation visant à produire des alpha-oléfines utilisées comme co-monomère des procédés de production de polyéthylène. La réaction d'oligomérisation est couramment réalisée dans un procédé de catalyse homogène en phase liquide dans un réacteur bi-phasique gaz/liquide, en général avec une mise en oeuvre dans une colonne à bulles, ou dans un réacteur monophasique liquide.

La réaction d'oligomérisation est fortement exothermique, il est courant de réguler la température de réaction par la mise en oeuvre d'un refroidissement externe. Généralement, un réacteur est couplé à une ou plusieurs boucle(s) de recirculation afin de soutirer une fraction liquide, de la refroidir par un ou des échangeurs, et de la réintroduire dans le réacteur. Ladite boucle de recirculation permet d'obtenir une bonne homogénéité des concentrations et de contrôler la température dans l'ensemble du volume réactionnel. Le brevet EP2703373 propose un procédé de trimérisation de l'éthylène en hexène-1 permettant de diminuer le coût des installations en limitant la consommation énergétique liée à la boucle de recirculation. Pour cela, une fraction de fond composée principalement de solvant issue de la section de séparation est utilisée dans l'échangeur thermique de la boucle de recirculation et également pour le rebouillage du fond d'une colonne de la section de séparation. US 2020/ 001266 divulgue un procédé avec une boucle de recirculation comprenant des moyens de soutirage dans un réacteur gaz/liquide pour le soutirage et l'envoi d'une fraction de liquide soutiré vers un échangeur thermique apte au refroidissement de ladite fraction liquide, et des moyens d'introduction dudit liquide refroidi, sortant de l'échangeur thermique, dans la partie supérieure du réacteur gaz/liquide.

Dans le domaine de l'invention, l'homme du métier cherche constamment à maîtriser le dimensionnement des équipements ayant un impact sur les performances et le coût du procédé et à réduire le coût des installations mises en oeuvre pour réaliser l'oligomérisation.

La demanderesse a découvert un procédé d'oligomérisation d'une charge oléfinique caractérisé en ce qu'une fraction solvant issue d'une étape aval de séparation est refroidie et recyclée de manière à contrôler en partie l'exothermie générée par la réaction d'oligomérisation dans le réacteur. Le procédé selon l'invention permet de limiter la taille du ou des échangeurs de chaleur utilisés dans au moins une boucle de recirculation par la mise en oeuvre du recyclage d'une fraction refroidie de solvant issue d'une section de séparation. La présente invention a pour but d'améliorer le procédé d'oligomérisation d'une charge oléfinique, en particulier de l'éthylène, dans un réacteur gaz/liquide ou dans un réacteur tout liquide. Elle cherche notamment à améliorer la productivité/la rentabilité du procédé, notamment afin d'éviter le phénomène de perçage et/ou afin de limiter les coûts d'investissement et/ou d'exploitation du procédé.

### Objet de l'invention

La présente invention porte sur un procédé d'oligomérisation d'une charge oléfinique mis en oeuvre à une température comprise entre 30 et 200°C et une pression comprise entre 0,1 et 10 MPa, en présence d'un système catalytique d'oligomérisation homogène et d'un solvant comprenant
a) Une étape d'oligomérisation de ladite charge oléfinique dans une section réactionnelle comprenant un réacteur d'oligomérisation et une ou plusieurs boucles de recirculation permettant le contrôle de la température dans ledit réacteur par le refroidissement d'une fraction de phase liquide,
b) Une étape de séparation dans une section aval de séparation d'un effluent de réaction issu de l'étape a) oligomérisation de façon à obtenir une fraction solvant,
c) Une étape de refroidissement de la fraction solvant issue de l'étape b) à une température inférieure à la température de la ou des boucle(s) de recirculation,
d) Une étape d'introduction à l'étape a) d'oligomérisation de la fraction solvant refroidie issue de l'étape c) dans la section réactionnelle.

De préférence, la fraction solvant issue de l'étape b) est refroidie à une température comprise entre 0 et 150°C.

De préférence, la fraction solvant issue de l'étape b) est refroidie à l'étape c) à une température inférieure d'au moins 40°C par rapport à la température de la fraction liquide refroidie dans la boucle de recirculation.

De préférence, le refroidissement de la fraction solvant à l'étape c) est réalisé par un ou plusieurs échangeur(s) thermique(s), de préférence choisi(s) parmi un ou plusieurs échangeurs de chaleur du type fluide process/fluide process, de type aéroréfrigérant, de type échangeur à eau de refroidissement.

De préférence, la section aval de séparation comprend au moins deux colonnes de distillation, de préférence au moins trois colonnes de distillation, de préférence au moins quatre colonnes de distillation.

De préférence, l'étape d) d'introduction de la fraction solvant refroidie est réalisée dans le réacteur et/ou dans une ou plusieurs des boucle(s) de recirculation.

De préférence, l'étape d) d'introduction de la fraction refroidie est réalisée dans une boucle de recirculation en amont ou en aval d'un échangeur thermique de ladite boucle de recirculation, de préférence en aval dudit échangeur thermique.

De préférence, lequel la fraction solvant refroidie présente un débit en un pourcentage massique par rapport au débit du liquide circulant dans la ou les boucle()s de recirculation compris entre 0,05 et 15,0%, de préférence entre 0,1 et 10,0%.

De préférence, la charge oléfinique comprend des oléfines ayant entre 2 et 6 atomes de carbone, de préférence entre 2 et 4 atomes de carbone. Avantageusement, la charge oléfinique est choisie parmi le butène, le propylène, et l'éthylène, seul ou en mélange.

De préférence, lequel l'étape a) d'oligomérisation comprend au moins une des sous-étapes suivantes
- sous-étape a1) d'introduction du système catalytique,
- sous-étape a2) de mise en contact avec de la charge oléfinique,
- sous-étape étape a3) de soutirage d'une fraction de phase liquide du réacteur d'oligomérisation,
- sous-étape a4) de refroidissement d'au moins une partie de la fraction liquide soutirée à l'étape a3),
- sous-étape a5) d'introduction dans le réacteur de la fraction liquide refroidie.

De préférence, la sous étape a4) de refroidissement est mise en oeuvre par la circulation d'au moins une partie de la fraction liquide soutirée à l'étape a3), au travers d'un ou plusieurs échangeurs thermiques situés dans la boucle de recirculation.

Avantageusement, le ou les échangeurs thermiques mis en oeuvre à la sous-étape a4) diminue la température de la fraction liquide de 1,0 à 30,0°C, de préférence entre 2,0 et 25,0°C.

De préférence, l'effluent de réaction est obtenu par division en deux flux de la fraction liquide soutirée à l'étape a3).

De préférence, le réacteur d'oligomérisation est choisi parmi un réacteur biphasique gaz/liquide ou monophasique liquide, de manière préférée est un réacteur biphasique gaz/liquide, de manière très préférée de type colonnes à bulles.

### Définition

Dans le cadre de la présente invention, le terme « section de fractionnement » désigne le ou les dispositifs de séparation, notamment par distillation, disposés en aval de la section réactionnelle, avec un seul dispositif ou une pluralité de dispositifs disposés en série et/ou en parallèle, dispositifs qui peuvent être identiques ou différents par leur dimensionnement ou leur conception/fonctionnement.

Dans le cadre du présent texte, on comprend les termes « amont » et « aval » en fonction du sens général d'écoulement du fluide réactionnel dans l'unité de production.

Dans le cadre du présent texte, les expressions échangeur de chaleur et échangeur thermique sont utilisées de façon équivalente.

On entend par catalyseur ou système catalytique homogène, le fait que le catalyseur ou le système catalytique est dans la même phase que les réactifs et les produits de la réaction d'oligomérisation.

On entend par perçage le passage d'éthylène gazeux de la phase liquide vers la phase gazeuse contenu dans un réacteur gal/liquide.

On désigne par taux de solvant, le ratio massique du débit total de solvant injecté sur la somme du débit total d'éthylène gazeux injecté et du débit de solvant injecté dans le réacteur.

On entend par réacteur biphasique gaz/liquide un réacteur comprenant une phase liquide dans une zone inférieure et une phase gazeuse dans une zone supérieure lorsqu'il est mis en oeuvre dans un procédé d'oligomérisation.

Dans le cadre de la présente invention, le terme « section réactionnelle » désigne le réacteur et la ou les boucle(s) de recirculation.

On entend par phase liquide, le mélange de l'ensemble des composés qui se trouvent à un état physique liquide dans les conditions de température et de pression du réacteur.

On entend par zone inférieure du réacteur, la partie du réacteur comprenant la phase liquide, la charge oléfinique gazeuse, en particulier de l'éthylène gazeux, les produits de la réaction tels que l'alpha oléfine linéaire souhaitée (*i*.*e*. butène-1, hexène-1, octène-1 ou le mélange d'alpha-oléfines linéaires), le système catalytique et un solvant.

On entend par zone supérieure du réacteur, la partie du réacteur se situant au sommet de l'enceinte, c'est-à-dire directement au-dessus de la zone inférieure et constituée de la phase gazeuse correspondant au ciel gazeux.

On entend par partie inférieure du réacteur, le quart inférieur dudit réacteur contenant la phase liquide.

On entend par partie supérieure du réacteur, le quart supérieur dudit réacteur contenant la phase liquide.

On entend par volume de liquide réactionnel, la quantité de liquide dans lequel se produit la réaction d'oligomérisation contenue dans le réacteur et/ou la ou les boucles de recirculation.

On désigne par volume d'une ou des boucles de recirculation, la taille de la ou desdites boucles correspondant au volume de liquide réactionnel pouvant être contenu par la ou desdites boucles.

### Procédé

La présente invention concerne un procédé d'oligomérisation d'une charge oléfinique dans lequel une fraction solvant issue d'une étape aval de séparation est refroidie et recyclée à la section réactionnelle de manière à contrôler en partie l'exothermie de l'étape d'oligomérisation.

En particulier, la présente invention concerne un procédé d'oligomérisation d'une charge oléfinique mis en oeuvre à une température comprise entre 30 et 200°C et une pression comprise entre 0,1 et 10 MPa, en présence d'un système catalytique d'oligomérisation homogène et d'un solvant comprenant
a) Une étape d'oligomérisation dans une section réactionnelle comprenant un réacteur d'oligomérisation et au moins une boucle de recirculation permettant le contrôle de la température dans ledit réacteur par le refroidissement d'une fraction de phase liquide,
b) Une étape de séparation dans une section aval de séparation d'un effluent issu de l'étape a) oligomérisation de façon à obtenir une fraction solvant,
c) Une étape de refroidissement de la fraction solvant issue de l'étape b) à une température inférieure à la température de la boucle de recirculation,
d) Une étape d'introduction à l'étape a) d'oligomérisation de la fraction solvant refroidie issue de l'étape c).

L'invention permet de réduire l'échange de chaleur au niveau de la boucle de recirculation tout en minimisant la température du solvant introduit dans le réacteur par la boucle de recirculation. Le contrôle de la température par la fraction solvant refroidie issue de la séparation aval permet de réduire la quantité de calories à échanger au niveau des boucles de recirculation et donc la taille des échangeurs. Le gain attendu peut être avantageusement de l'ordre de 1 à 50%, de préférence entre 2% et 40% et préférentiellement entre 3 et 30% sur la surface d'échange.

Par ailleurs, la réaction d'oligomérisation a lieu à la fois dans le liquide réactionnel contenu dans le réacteur et dans la ou les boucle(s) de recirculation. La réduction de la taille des échangeurs de chaleur signifie que le volume d'au moins une boucle de recirculation diminue.

Lorsque le procédé selon l'invention est mis en oeuvre avec un volume de liquide réactionnel total (réacteur + boucle de recirculation) identique par rapport à un procédé ne mettant pas en oeuvre l'invention, alors le volume de liquide réactionnel dans le réacteur augmente ce qui permet d'améliorer la saturation du milieu réactionnel liquide en charge oléfinique et donc les performances du procédé.

Lorsque le procédé selon l'invention est mis en oeuvre avec une quantité de liquide réactionnel dans le réacteur identique par rapport à un procédé ne mettant pas en oeuvre l'invention, alors la proportion de volume de liquide réactionnel dans une boucle de recirculation diminue par rapport au volume total de liquide réactionnel. Cette diminution permet de réduire le temps de séjour et donc d'améliorer les performances du systèmes catalytique en terme d'activité et de sélectivité.

Ainsi la présente invention permet de moduler facilement la productivité et la rentabilité du procédé d'oligomérisation en fonction des performances du système catalytique mis en oeuvre.

### Système catalytique d'oligomérisation homogène

Tous les systèmes catalytiques connus de l'Homme du métier et aptes à être mis en oeuvre dans les procédés de dimérisation, de trimérisation, de tétramérisation et plus généralement dans les procédés d'oligomérisation selon l'invention, font partie du domaine de l'invention. Lesdits systèmes catalytiques ainsi que leur mise en oeuvre sont notamment décrits dans les demandes FR2984311, FR2552079, FR3019064, FR3023183, FR3042989 ou encore dans la demande FR3045414.

De préférence, les systèmes catalytiques comprennent, de préférence sont constitués de :
- un précurseur métallique de préférence à base de nickel, de titane ou de chrome,
- optionnellement un agent activateur,
- optionnellement un additif, et
- optionnellement un solvant.

### Le précurseur métallique

Le précurseur métallique utilisé dans le système catalytique est choisi parmi les composés à base de nickel, de titane ou de chrome.

Dans un mode de réalisation, le précurseur métallique est à base de nickel et préférentiellement comprend du nickel de degré d'oxydation (+II). De préférence, le précurseur de nickel est choisi parmi les carboxylates de nickel(Il) tel que par exemple le 2-éthylhexanoate de nickel, les phénates de nickel(II), les naphténates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de π-allylnickel(II), le bromure de rr-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydratée ou non, pris seul ou en mélange.

Dans un second mode de réalisation, le précurseur métallique est à base de titane et préférentiellement comprend un composé aryloxy ou alcoxy du titane.

Le composé alcoxy du titane répond avantageusement à la formule générale [Ti(OR)₄] dans laquelle R est un radical alkyle linéaire ou ramifié. Parmi les radicaux alcoxy préférés, on peut citer à titre d'exemple non limitatifs : le tétraéthoxy, le tétraisopropoxy, le tétra-n-butoxy et le tétra-2-éthyl-hexyloxy.

Le composé aryloxy du titane répond avantageusement à la formule générale [Ti(OR')₄] dans laquelle R' est un radical aryle substitué ou non par des groupements alkyle ou aryle. Le radical R' peut comporter des substituants à base d'hétéroatome. Les radicaux aryloxy préférés sont choisis parmi le phénoxy, le 2-méthylphénoxy, le 2,6-diméthylphénoxy, le 2,4,6-triméthylphénoxy, le 4-méthylphénoxy, le 2-phénylphénoxy, le 2,6-diphénylphénoxy, le 2,4,6-triphénylphénoxy, le 4-phénylphénoxy, le 2-tert-butyl-6-phénylphénoxy, le 2,4-ditertbutyl-6-phénylphénoxy, le 2,6-diisopropylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy, le radical biphénoxy, le binaphtoxy, le 1,8-naphtalène-dioxy.

Selon un troisième mode de réalisation, le précurseur métallique est à base de chrome et préférentiellement comprend un sel de chrome (II), un sel de chrome (III), ou un sel de degré d'oxydation différent pouvant comporter un ou plusieurs anions identiques ou différents, tels que par exemple des halogénures, des carboxylates, des acétylacétonates, des anions alcoxy ou aryloxy. De préférence, le précurseur à base de chrome est choisi parmi CrCl₃, CrCl₃(tétrahydrofurane)₃, Cr(acétylacétonate)₃, Cr(naphténate)₃, Cr(2-éthylhexanoate)₃, Cr(acétate)₃.

La concentration en nickel, en titane ou en chrome, est comprise entre 0,001 et 300,0 ppm en masse de métal atomique par rapport à la masse réactionnelle, de préférence entre 0,002 et 100,0 ppm, préférentiellement entre 0,003 et 50,0 ppm, plus préférentiellement entre 0,05 et 20,0 ppm et encore plus préférentiellement entre 0,1 et 10,0 ppm en masse de métal atomique par rapport à la masse réactionnelle.

### L'agent activateur

Optionnellement, quel que soit le précurseur métallique, le système catalytique comprend un ou plusieurs agents activateurs choisis parmi les composés à base d'aluminium tels que le dichlorure de méthylaluminium (MeAlCl₂), le dichloroéthylaluminium (EtAlCl₂), le sesquichlorure d'éthylaluminium (Et₃Al₂Cl₃), le chlorodiéthylaluminium (Et₂AlCl), le chlorodiisobutylaluminium (i-Bu₂AlCl), le triéthylaluminium (AlEt₃), le tripropylaluminium (AI(n-Pr)₃), le triisobutylaluminium (Al(i-Bu)₃), le diéthyl-éthoxyaluminium (Et₂AlOEt), le méthylaluminoxane (MAO), l'éthylaluminoxane et les méthylaluminoxanes modifiés (MMAO).

### L'additif

Optionnellement, le système catalytique comprend un ou plusieurs additifs.

L'additif est choisi parmi les composés phosphorés monodentés, des composés phosphorés bidentés, des composés phosphorés tridentés, des composés oléfiniques, des composés aromatiques, des composés azotés, des bipyridines, des diimines, des éthers monodentés, des éthers bidentés, des thioéthers monodentés, des thioéthers bidentés, des carbènes monodentés ou bidentés, des ligands mixtes tels que des phosphinopyridines, des iminopyridines, des bis(imino)pyridines

Lorsque le système catalytique est à base de nickel, l'additif est choisi parmi,
- les composés de type azoté, tels que la triméthylamine, la triéthylamine, le pyrrole, le 2,5-diméthylpyrrole, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1 ,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, ou
- les composés de type phosphine choisi indépendamment parmi la tributylphosphine, la triisopropylphosphine, la tricyclopentylphosphine, la tricyclohexylphosphine, la triphénylphosphine, la tris(o-tolyl)phosphine, le bis(diphénylphosphino)éthane, l'oxyde de trioctylphosphine, l'oxyde de triphénylphosphine, la triphénylphosphite, ou
- les composés répondant à la formule générale (I) ou un des tautomères dudit composé : 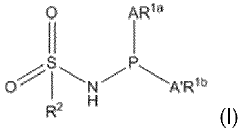 dans laquelle
   - A et A', identiques ou différents, sont indépendamment un oxygène ou une liaison simple entre l'atome de phosphore et un atome de carbone,
   - les groupements R^{1a} et R^{1b} sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 2-méthylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle,
   - le groupement R² est choisi indépendamment parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-
   diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle.

Lorsque le système catalytique est à base de titane, l'additif est choisi parmi l'éther diéthylique, le diisopropyléther, le dibutyléther, le diphényléther, le 2-méthoxy-2-méthylpropane, 2-methoxy-2-méthylbutane, le diméthoxy-2,2 propane, le di(2-éthylhexyloxy)-2,2 propane, le 2,5-dihydrofurane, le tétrahydrofurane, le 2-méthoxytétrahydrofurane, le 2-méthyltétrahydrofurane, le 3-méthyltétrahydrofurane, le 2,3-dihydropyrane, le tétrahydropyrane, le 1,3-dioxolane, le 1,3-dioxane, le 1,4-dioxane, le diméthoxyéthane, di(2-méthoxyéthyl)éther, le benzofurane, le glyme et le diglyme pris seuls ou en mélange.

Lorsque le système catalytique est à base de chrome, l'additif est choisi parmi,
- les composés de type azoté, tels que la triméthylamine, la triéthylamine, le pyrrole, le 2,5-diméthylpyrrole, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, ou
- les composés aryloxy de formule générale [M(R³O)₂₋ₙXₙ]_{y} dans laquelle
   * M est choisi parmi le magnésium, le calcium, le strontium et le baryum, de préférence le magnésium,
   * R³ est un radical aryl contenant de 6 à 30 atomes de carbone, X est un halogène ou un radical alkyl contenant de 1 à 20 atomes de carbone,
   * n est un nombre entier qui peut prendre les valeurs de 0 ou 1, et
   * y est un nombre entier compris entre 1 et 10, de préférence y est égal à 1, 2, 3 ou 4.

De préférence, le radical aryloxy R³O est choisi parmi le 4-phénylphénoxy, le 2-phénylphénoxy, le 2,6-diphénylphénoxy, le 2,4,6-triphénylphénoxy, le 2,3,5,6-tétraphénylphénoxy, le 2-tert-butyl-6-phénylphénoxy, le 2,4-ditertbutyl-6-phénylphénoxy, le 2,6-diisopropylphénoxy, le 2,6-diméthylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy. Les deux radicaux aryloxy peuvent être portés par une même molécule, comme par exemple le radical biphénoxy, le binaphtoxy ou le 1,8-naphtalène-dioxy, De préférence, le radical aryloxy R³O est le 2,6-diphénylphénoxy, le 2-tert-butyl-6-phénylphénoxy ou le 2,4-ditert-butyl-6-phénylphénoxy.

### Etape a) d'oligomérisation

Le procédé selon l'invention comprend donc une étape a) d'oligomérisation de la charge oléfinique mise en oeuvre à une température comprise entre 30 et 200°C et une pression comprise entre 0,1 et 10 MPa, en présence d'un système catalytique d'oligomérisation homogène et d'un solvant. Ladite étape a) d'oligomérisation est réalisée dans une section réactionnelle comprenant un réacteur d'oligomérisation et au moins une boucle de recirculation permettant le contrôle de la température dans ledit réacteur par le refroidissement d'une fraction de phase liquide. Le refroidissement de la fraction de liquide consiste à refroidir ladite fraction à une température inférieure à celle de la température d'oligomérisation de manière à contrôler l'exothermie de la réaction.

De préférence, le réacteur d'oligomérisation est choisi parmi un réacteur biphasique gaz/liquide ou monophasique liquide, de manière préférée est un réacteur biphasique gaz/liquide, de manière très préférée est un réacteur de type colonne à bulles.

La charge oléfinique comprend de préférence des oléfines ayant entre 2 et 6 atomes de carbone, de préférence entre 2 et 4 atomes de carbone. De préférence, la charge oléfinique est choisie parmi le butène, plus particulièrement l'isobutène ou le butène-1, le propylène, et l'éthylène, seul ou en mélange.

Dans le reste du présent texte, sauf mention contraire, quand on mentionne spécifiquement l'éthylène, on désigne également les oléfines ayant entre 2 et 6 atomes de carbone, donc l'isobutène ou le butène-1, le propylène, et l'éthylène.

De préférence, le procédé d'oligomérisation est un procédé de dimérisation, de trimérisation ou de tétramérisation, de préférence de l'éthylène.

Avantageusement, le procédé d'oligomérisation est mis en oeuvre à une pression comprise entre 0,1 et 10,0 MPa, de préférence entre 0,2 et 9,0 MPa et préférentiellement entre 0,3 et 8,0 MPa, à une température comprise entre 30 et 200°C, de préférence entre 35 et 180°C, de préférence entre 45 et 170°C, de préférence entre 60 et 160°C, de préférence entre 70 et 150°C, de préférence entre 80 et 145°C et de manière préférée entre 100 et 140°C.

L'effluent de réaction issu de l'étape a) envoyé vers la section de séparation aval de la section réactionnelle est obtenu par soutirage d'une fraction liquide du réacteur.

Dans un mode de réalisation particulier, l'effluent de réaction est obtenu par division en deux flux de la fraction liquide soutirée à l'étape a3) définie ci-après. Le premier flux est envoyé vers l'étape a4) de refroidissement, et le second flux correspond à l'effluent et est envoyé vers la section aval de séparation.

Avantageusement, le débit de l'effluent de réaction est régulé pour maintenir un niveau liquide constant dans le réacteur. De préférence, le débit dudit effluent est de 5 à 200 fois inférieur au débit liquide envoyé à l'étape a4) de refroidissement. De préférence, le débit dudit effluent est de 5 à 150 fois inférieur, de préférence de 10 à 120 fois inférieur et de manière préférée de 20 à 100 fois inférieur.

La réaction d'oligomérisation ayant lieu à la fois dans le réacteur et dans la ou les boucle(s) de recirculation, le temps de séjour dans la section réactionnelle s'entend donc sur l'ensemble du volume du réacteur et de la ou des boucle(s) de recirculation composant la section réactionnelle.

Avantageusement, le procédé d'oligomérisation est mis en oeuvre avec un taux de solvant compris entre 0 et 90% pds, de préférence entre à 10 et 85% pds, de préférence entre 20 et 80% pds, de préférence entre 30 et 75% pds. Avantageusement, le procédé d'oligomérisation est mis en oeuvre dans un réacteur biphasique gaz/liquide de type colonne à bulles par exemple avec une charge éthylène gazeux. La dissolution des bulles d'éthylène dans le milieu réactionnel a lieu dans la colonne à bulles. Plus la hauteur de liquide disponible dans cette colonne est importante plus la dissolution de l'éthylène est proche de la complète saturation. La sélectivité de la réaction vers le produit principal de réaction étant inversement liée à la conversion de l'éthylène dans le liquide, maximiser la quantité d'éthylène dissous permettra à débit d'éthylène constant en entrée du réacteur de diminuer la conversion et donc d'augmenter la sélectivité.

Les boucles de recirculation représentant une proportion importante du volume de liquide réactionnel de la section réactionnel, il est d'intérêt de réduire au maximum ce volume afin
- Soit d'augmenter le volume de liquide réactionnel dans le réacteur (par exemple dans une colonne à bulles) et en conséquence la hauteur liquide dans le réacteur, en particulier dans la colonne à bulle et ainsi de maximiser la saturation en éthylène dans le liquide,
- Soit de diminuer le volume de la ou les boucles de recirculation tout en maintenant un volume du réacteur constant et ainsi réduire le temps de séjour ce qui peut permettre d'améliorer les performances du système catalytique en terme d'activité et de sélectivité.

Le ou les solvants sont avantageusement choisis parmi les éthers, les alcools, les solvants halogénés et les hydrocarbures, saturés ou insaturés, cycliques ou non, aromatiques ou non, comprenant entre 1 et 20 atomes de carbone, de préférence entre 4 et 15 atomes de carbone, préférentiellement entre 4 et 12 atomes de carbone et encore plus préférentiellement entre 4 et 8 atomes de carbone.

De préférence, le solvant est choisi parmi le pentane, l'hexane, le cyclohexane, le méthylcyclohexane, l'heptane, le butane ou l'isobutane, le cycloocta-1,5-diène, le benzène, le toluène, l'ortho-xylène, le mésitylène, l'éthylbenzène, le diéthyléther, le tétrahydrofurane, le 1,4-dioxane, le dichlorométhane, le dichloroéthane, le tétrachloroéthane, l'hexachloroéthane, le chlorobenzène, le dichlorobenzène, le butène, l'hexène et l'octène purs ou en mélange.

De préférence, le solvant peut être avantageusement choisi parmi les produits de la réaction d'oligomérisation. De manière préférée, le solvant utilisé est le cyclohexane.

Afin d'évacuer l'énergie de la réaction, une ou plusieurs boucle(s) de recirculation sont utilisées. La boucle de recirculation permet de faire circuler depuis le fond du réacteur, une fraction de phase liquide comprenant les produits de la réaction, le solvant et le système catalytique au travers d'un échangeur avant d'être renvoyée en tête du réacteur.

De préférence, les alpha oléfines linéaires obtenues comprennent de 4 à 20 atomes de carbone, de préférence de 4 à 18 atomes de carbone, de préférence de 4 à 10 atomes de carbone, et de préférence de 4 à 8 atomes de carbone. De manière préférée, les oléfines sont des alpha-oléfines linéaires, choisi parmi le but-1-ène, le hex-1-ène ou l'oct-1-ène.

Avantageusement, la section réactionnelle comprend un ou plusieurs réacteurs de type gaz/liquide ou tout liquide, disposé en série et/ou en parallèle, ainsi que leurs équipements associés tels que :
- une ou des boucles de recirculation comprenant un ou plusieurs échangeurs thermiques et associées au/à chacun des réacteurs pour contrôler l'exothermie de la réaction,
- des moyens d'introduction du système catalytique d'oligomérisation dans le ou les sections réactionnelles,
- des moyens externes à la section réactionnelle pour séparer/neutraliser le système catalytique.

Avantageusement, l'étape a) d'oligomérisation comprend au moins une des sous-étapes suivantes
- sous-étape a1) d'introduction du système catalytique,
- sous-étape a2) de mise en contact avec de la charge oléfinique,
- sous-étape a3) de soutirage d'une fraction de phase liquide,
- sous-étape a4) de refroidissement de la fraction liquide,
- sous-étape a5) d'introduction dans le réacteur de la fraction liquide refroidie.

De préférence, l'étape a) d'oligomérisation comprend les sous-étapes a1), a2), a3), a4) et a5).

### Sous-étape a1) d'introduction du système catalytique

Avantageusement l'étape d'oligomérisation a) comprend une sous-étape a1) d'introduction d'un système catalytique comprenant un précurseur métallique et un agent activateur, éventuellement d'un additif et éventuellement d'un solvant ou d'un mélange de solvants, de préférence dans un réacteur biphasique gaz/liquide ou monophasique liquide.

Lorsque le procédé d'oligomérisation met en oeuvre un réacteur biphasique gaz/liquide, ledit réacteur comprend une phase liquide dans une zone inférieure et une phase gazeuse dans une zone supérieure.

De préférence, l'introduction du système catalytique est réalisée en mélange avec la fraction liquide introduite à l'étape a5).

De préférence, la pression d'introduction dans le réacteur est comprise entre 0,1 et 10,0 MPa, de préférence entre 0,2 et 9,0 MPa et préférentiellement entre 0,3 et 8,0 MPa.

### Sous-étape a2) de mise en contact avec la charge oléfinique

Avantageusement l'étape d'oligomérisation a) comprend une sous-étape a2) d'introduction de la charge oléfinique, de préférence de l'éthylène gazeux. De préférence, ladite charge oléfinique est introduite dans la phase liquide au niveau de la partie inférieure du réacteur. La charge oléfinique peut comprendre de la charge fraiche, et de préférence, en mélange avec de la charge oléfinique recyclée d'une étape de séparation aval au procédé d'oligomérisation.

De préférence, lorsque la charge oléfinique introduite est gazeuse, ladite charge est distribué par dispersion lors de son introduction dans la phase liquide inférieure du réacteur par un moyen apte à réaliser ladite dispersion de manière uniforme sur toute la section du réacteur. De préférence, le moyen de dispersion est choisi parmi un réseau distributeur avec une répartition homogène des points d'injection d'éthylène sur toute la section du réacteur.

De préférence, la charge oléfinique est introduite à un débit compris entre 1 et 250 t/h, de préférence entre 2 et 200 t/hde préférence entre 5 et 100 t/h.

De préférence, le débit de charge oléfinique introduite à l'étape a2) est asservi à la pression dans le réacteur.

Selon un mode particulier de mise en oeuvre de l'invention, un flux d'hydrogène gazeux peut également être introduit dans le réacteur, avec un débit représentant 0,01 à 1,0 % en masse du débit de charge oléfinique entrant. De préférence, le flux d'hydrogène gazeux est introduit par la conduite mise en oeuvre pour l'introduction de la charge oléfinique.

### Sous-étape a3) de soutirage d'une fraction de phase liquide

Avantageusement l'étape d'oligomérisation a) comprend une sous-étape étape a3) de soutirage d'une fraction de phase liquide du réacteur d'oligomérisation de préférence dans la partie inférieure dudit réacteur.

Le soutirage mis en oeuvre à l'étape a3) est, de préférence, réalisé sous le niveau de l'injection de la charge oléfinique, et de préférence dans le fond de l'enceinte. Le soutirage est mis en oeuvre par tout moyen apte à réaliser le soutirage et de préférence au moyen d'une conduite combinée à une pompe.

De préférence, le débit de soutirage est compris entre 10 et 10000 t/h, et de préférence entre 100 et 7000 t/h.

### Sous-étape a4) de refroidissement de la fraction liquide

Avantageusement, l'étape d'oligomérisation a) comprend une sous-étape a4) de refroidissement d'au moins une partie de la fraction liquide soutirée à l'étape a3). De préférence, l'étape de refroidissement est mise en oeuvre par la circulation d'au moins une partie de la fraction liquide soutirée à l'étape a3), au travers un ou plusieurs échangeurs thermiques situés dans la boucle de recirculation.

Avantageusement, le ou les échangeurs de chaleur mis en oeuvre à la sous étape a4) permettent de diminuer la température de la fraction liquide de 1,0 à 30,0°C, de préférence entre 2,0 et 25°C, de préférence entre 3,0 et 20,0°C et de manière préférée entre 5,0 et 15,0°C. Avantageusement le refroidissement de la fraction liquide permet de maintenir la température du milieu réactionnel dans les gammes de température souhaitées pour réaliser la réaction d'oligomérisation au sein du réacteur.

Avantageusement, la mise en oeuvre de l'étape de refroidissement du liquide, par l'intermédiaire de la boucle de recirculation permet également d'effectuer l'agitation du milieu réactionnel, et ainsi d'homogénéiser les concentrations des espèces réactives dans tout le volume liquide du réacteur.

### Sous-étape a5) d'introduction de la fraction liquide refroidie

Avantageusement l'étape d'oligomérisation a) comprend une sous-étape a5) d'introduction de la fraction liquide refroidie à l'étape a4).

L'introduction de la fraction liquide refroidie issue de l'étape a4) est, de préférence, réalisée dans la phase liquide du réacteur, de préférence dans la partie supérieure dudit réacteur, par tout moyen connu de l'Homme du métier, tel qu'une conduite.

De préférence, le débit d'introduction de la fraction liquide refroidie est compris entre 10 et 10000 t/h, et de préférence entre 100 et 7000 t/h.

Les sous étapes a3) à a5) constituent une boucle de recirculation. Avantageusement, la boucle de recirculation permet également d'assurer l'agitation du milieu réactionnel, et ainsi d'homogénéiser les concentrations des espèces réactives dans tout le volume liquide du réacteur.

### Etape b) de séparation aval

Le procédé selon l'invention comprend donc une étape b) de séparation aval d'un effluent issu de l'étape a) d'oligomérisation dans une section aval de façon à obtenir entre autre une fraction solvant.

Ladite fraction solvant est principalement composée de solvant. Avantageusement, la teneur en solvant de ladite fraction est supérieure ou égale à 95% poids, de préférence supérieure à 98% poids et de manière préférée supérieure ou égale à 99% poids.

Typiquement, l'étape de séparation aval peut être réalisée avec des moyens de séparation, tels que des colonnes de distillation, fonctionnant en série et basées sur les différences de points d'ébullition des composés à séparer. Les composés à séparer comprennent le ou les produits de la réaction d'oligomérisation tels que les alpha oléfines linéaires obtenues, éventuellement la charge oléfinique n'ayant pas réagi, et le ou les solvants.

De préférence, l'étape de séparation aval inclut également une étape préliminaire de neutralisation du catalyseur. Le catalyseur peut être retiré des produits de la réaction de manière dédiée ou en mélange avec les composés les plus lourds.

De préférence, la section aval de séparation comprend au moins deux colonnes de distillation, de préférence au moins trois colonnes de distillation, de préférence au moins quatre colonnes de distillation. Lesdites colonnes sont positionnées en parallèle et/ou en série, de préférence en série. Selon une variante préférée, la section de distillation comprend trois colonnes de distillation. La séparation de la fraction solvant peut être réalisée dans l'une quelconque des colonnes de distillation de la section aval de séparation dans la mesure où ladite fraction solvant est principalement composée de solvant afin de pouvoir recycler ladite fraction vers le réacteur d'oligomérisation.

De manière avantageuse, l'étape b) de séparation met en oeuvre une première colonne de distillation à une pression comprise entre 0,1 et 3,0 MPa, de préférence entre 0,5 et 1 MPa, une température de tête de colonne comprise entre 0 et 100°C, de préférence entre 40 et 80°C, et une température de fond de colonne comprise entre 100 et 300°C, de préférence entre 140 et 220°C. Ladite première colonne de distillation permet de séparer la charge oléfinique non convertie, en particulier l'éthylène non converti, dans une fraction de tête du reste des composés dans la fraction de fond.

De manière avantageuse, l'étape b) de séparation met en oeuvre une deuxième colonne de distillation à une pression comprise entre 0 et 2,0 MPa, de préférence entre 0,01 et 1,0 MPa, une température de tête de colonne comprise entre 20 et 150°C, de préférence entre 40 et 130°C, et une température de fond de colonne comprise entre 50 et 300°C, de préférence entre 80 et 250 °C. De manière préférée, ladite deuxième colonne permet de séparer de ladite fraction de fond issue de la première colonne en une fraction de tête (31) comprenant les alpha oléfines linéaires obtenues, en particulier de l'hexène-1, le solvant et une fraction de fond comprenant les composés les plus lourds.

De manière avantageuse, l'étape b) de séparation met en oeuvre une troisième colonne de distillation à une pression comprise entre 0 et 1,0 MPa, de préférence entre 0,01 et 0,5 MPa, une température de tête de colonne comprise entre 30 et 130°C, de préférence entre 50 et 90°C, et une température de fond de colonne comprise entre 50 et 200°C, de préférence entre 90 et 180°C. De manière préférée, ladite troisième colonne permet de séparer l'hexène-1 en tête du solvant en fond.

A titre d'exemple non limitatif, dans le cas de la trimérisation de l'éthylène en hexène-1, l'effluent issu de l'étape a) de trimérisation de l'éthylène comprenant, de l'éthylène, le solvant, le système catalytique de la trimérisation de l'éthylène et les produits formés dont l'hexène-1, peut être séparé dans un étape b) de séparation comprenant au moins les sous-étapes suivantes :
b1) une première étape de séparation dans une première colonne de distillation, de l'effluent de la réaction de trimérisation de l'éthylène, en une fraction de tête comprenant l'éthylène et une fraction de fond,
b2) une deuxième étape de séparation dans au moins une autre colonne de distillation au moins une partie de la fraction de fond issue de l'étape b1) en une fraction de tête comprenant de l'hexène-1 et le solvant et une fraction de fond comprenant des hydrocarbures C8+,
b3) une troisième étape de séparation dans une colonne de distillation finale au moins une partie de la fraction comprenant de l'hexène-1 et du solvant issue de l'étape b2) en une fraction de tête comprenant principalement de l'hexène-1 et en une fraction de fond comprenant principalement le solvant.

Conformément à l'invention, au moins une fraction solvant provenant de la fraction de fond issue de l'étape b3) est refroidie à l'étape c) puis renvoyée dans la section réactionnelle à l'étape d).

### Etape c) de refroidissement

Le procédé selon l'invention comprend une étape c) de refroidissement de la fraction solvant issue de l'étape b) de séparation aval à une température inférieure à la température de la boucle de recirculation.

Le refroidissement de la fraction solvant à une température inférieure à la température de la boucle de recirculation, de préférence de la température obtenue à l'issu de l'étape a4), permet de diminuer l'échange de chaleur nécessaire dans la ou les boucles de recirculation pour atteindre la température de la fraction liquide refroidie issue de ladite boucle qui est introduite dans le réacteur, et donc de diminuer la taille du ou des échangeurs.

Le refroidissement de la fraction solvant à l'étape c) peut être réalisé par un ou plusieurs échangeur thermique échangeant soit avec un fluide du procédé, soit avec de l'air, soit avec de l'eau de refroidissement ou tout autre type de fluide froid permettant d'atteindre la température souhaitée ou à l'aide d'une combinaison de ces échangeurs. Avantageusement, l'échangeur est choisi parmi un ou plusieurs échangeurs de chaleur du type fluide process/fluide process (de type TEMA ou autres connus de l'homme du métier), de type aéroréfrigérant, de type échangeur à eau de refroidissement ou tout autre type de fluide froid permettant d'atteindre la température souhaitée.

Avantageusement, la fraction solvant issue de l'étape b) est refroidie à une température comprise entre 0 et 150°C, de préférence entre à 5 et 100°C, de préférence entre 10 et 90°C, de préférence entre 20 et 80°C, de préférence entre 25 et 70°C, et de manière préférée entre 30 et 60°C.

Avantageusement, la fraction solvant issue de l'étape b) est refroidie à l'étape c) à une température inférieure d'au moins 40°C, de préférence d'au moins 50°C, de préférence d'au moins 60°C, de préférence d'au moins 70°C par rapport à la température souhaitée de la fraction liquide refroidie dans la boucle de recirculation de la section réactionnelle.

### Etape d) d'introduction de la fraction issue de c)

Le procédé selon l'invention comprend donc une étape d) d'introduction à l'étape a) d'oligomérisation de la fraction solvant refroidi à l'étape c) à une température inférieure à la température de la boucle de recirculation.

Avantageusement, l'introduction de la fraction solvant refroidie est réalisée dans la section réactionnelle, de préférence dans le réacteur et/ou dans une ou plusieurs des boucle(s) de recirculation. De préférence, l'introduction est réalisée dans une boucle de recirculation en amont ou en aval de l'échangeur thermique de ladite boucle de recirculation, c'est-à-dire en amont ou en en aval de l'étape a4), de manière préférée l'introduction est réalisée en aval de l'échangeur thermique.

L'introduction de la fraction solvant refroidie selon l'invention permet de contrôler en partie l'exothermie de l'étape d'oligomérisation et ainsi de limiter la taille du ou des échangeurs de chaleur mis en oeuvre dans au moins une boucle de recirculation

Avantageusement, l'introduction de la fraction solvant refroidie en aval de l'échangeur thermique de la boucle de recirculation permet de minimiser l'écart de température entre la fraction solvant refroidie et la fraction liquide de la boucle de recirculation. Cela permet également de maximiser l'utilisation de l'échangeur de la ou des boucles de recirculation et donc de minimiser sa taille.

Avantageusement, le mélange de la fraction solvant refroidie à l'étape c) avec la fraction liquide circulant dans la boucle de recirculation de la section réactionnelle permet de diminuer la température de la fraction liquide de la boucle de recirculation de 0,1 à 20,0°C, de préférence entre 0,2 et 15,0°C et de manière préférée entre 0,5 et 10,0°C.

Avantageusement, la fraction solvant refroidie présente un débit en un pourcentage massique par rapport au débit du liquide circulant dans la boucle de recirculation compris entre 0,05 et 15,0%, de préférence entre 0,1 et 10,0%, de préférence entre 0,5 et 8,0%, de préférence entre 0,8 et 6,0% et de manière préférée entre 1,0 et 5,0%.

Ainsi la mise en oeuvre des étapes c) et d) selon l'invention permet par l'intermédiaire de la de la fraction solvant refroidie de réduire l'énergie échangée au niveau des boucles de recirculation, et donc la taille de l'échangeur. Le gain attendu peut être avantageusement de l'ordre de 1 à 50%, de préférence entre 2% et 30% et préférentiellement entre 3 et 20% sur la surface d'échange.

### Description de la figure

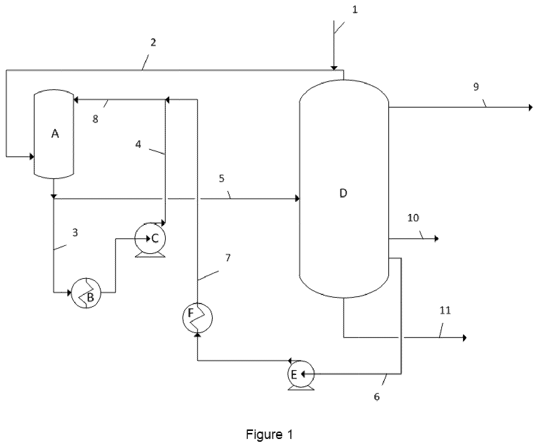

La figure 1 représente une illustration schématique d'une installation mettant en oeuvre un mode de réalisation du procédé d'oligomérisation selon l'invention. Ladite installation comprend un réacteur d'oligomérisation biphasique liquide/gaz A, une boucle de recirculation comprenant un échangeur B et une pompe C, une section de séparation D, une pompe E de circulation de la fraction solvant, un échangeur de refroidissement de la fraction solvant (F). Dans cette installation, le flux 2 est un mélange du flux 1 d'éthylène frais et d'éthylène issu de la section de séparation. Le flux 2 est introduit dans le réacteur A. Le flux 3 est la fraction de phase liquide soutirée du réacteur et envoyée dans la boucle de recirculation comprenant un échangeur B et une pompe C pour obtenir une fraction liquide refroidie 4. Le flux 6 est la fraction de solvant séparée dans la section de séparation D qui passe par une pompe E et est refroidie dans un échangeur F en une fraction solvant refroidie 7. Les fractions 7 et 4 sont mélangées en un flux 8 avant d'être introduit dans le réacteur A. Le flux 5 correspondant à une partie de l'effluent soutiré du réacteur A est envoyé vers la section de séparation D. La section de séparation permet d'obtenir le flux 6, un flux 9 correspondant aux produits de réaction légers, un flux 10 correspondant aux produits de réaction lourds et un flux 11 correspondant à une fraction lourde comprenant le catalyseur usé.

### Exemples

Les exemples ci-dessous illustrent l'invention sans en limiter la portée.

Les exemples ci-après décrivent un procédé d'oligomérisation de l'éthylène mis en oeuvre en continu dans un réacteur biphasique gaz/liquide de type colonne à bulles à une pression de 6,1 MPa et une température de 135°C. Le système catalytique est introduit dans le réacteur à une concentration de 1 ppm en poids de chrome, comprend le précurseur de chrome Cr(2-éthylhexanoate)₃, du 2-5-dimethylpyrrole à un ratio molaire par rapport au chrome de 3, 11 équivalents molaires de triéthylaluminium et 8 équivalents molaires de chlorure de diéthylaluminium par rapport au chrome en présence de o-xylène comme additif à un ratio molaire de 500 par rapport au chrome et de cyclohexane comme solvant.

La quantité de cyclohexane, utilisé comme solvant 6, introduit dans le réacteur A est dépendante de la quantité d'éthylène entrant dans le même réacteur A (flux 2), la quantité de solvant est ajustée de manière à avoir un taux de solvant de 58% dans le réacteur.

### Exemple 1 (comparatif)

L'exemple 1 illustre un mode de réalisation selon l'art antérieur de référence correspondant à la Figure 1, à l'exception que la fraction solvant (7) recyclé de la section de séparation vers la section réactionnelle n'est pas refroidie par un échangeur de chaleur F et dans lequel le procédé d'oligomérisation met en oeuvre un réacteur gaz-liquide de type colonne à bulle.

Le système catalytique est mis en contact avec de l'éthylène gazeux par introduction dudit éthylène gazeux dans la partie inférieure dudit réacteur. L'effluent est ensuite récupéré en fond de réacteur.

La production du produit valorisé nécessite la conversion de 14000 kg/h de réactif. Le débit de solvant dans les conditions d'opération retenues est de 19500 kg/h. La température de la fraction solvant recyclé est de 101°C.

Le temps de séjour dans la section réactionnelle (réacteurs + boucle(s) de recirculation) est de 40 minutes.

La réaction d'oligomérisation étant exothermique, la chaleur de la réaction est évacuée par des échangeurs de chaleur placés sur des boucles de recirculation au réacteur, de 1650 m² de surface totale. Le volume de liquide réactionnel total de 41,3 m³ est réparti entre le volume pris par les échangeurs de chaleurs et leur boucle de recirculation, et le réacteur. Le volume de liquide réactionnel total se décompose de la façon suivante : 30,1 m³ pour les boucles d'échange de chaleur, et 11,2 m³ pour le réacteur. La hauteur de liquide dans la colonne à bulles est alors de 5,0 m pour un diamètre de 1,7 m. La température du mélange 8 du solvant 7 et du fluide de recirculation 4 est alors de 120°C. La température du flux 4, correspondant à la sortie de l'échangeur B, est alors de 120.4°C

La production en hexène-1 est de 9,32 tonnes/heure, la sélectivité en hexène-1 est de 93,2% poids.

### Exemple 2 (selon l'invention)

Le procédé d'oligomérisation selon l'invention est mis en oeuvre dans les mêmes conditions que dans l'exemple 1. L'exemple 2 comprend en outre une étape de refroidissement de la fraction solvant de la section de séparation vers la section réactionnelle par un échangeur f, telle que décrite dans la figure 1. Ladite fraction solvant est refroidie à une température de 40°C.

L'étape de refroidissement de la fraction solvant permet de réduire le besoin d'échange dans les boucles de recirculation qui se traduit par une diminution de la surface des échangeurs. La surface totale des échangeurs des boucles de recirculation est alors limitée à 1440 m². Dans cet exemple, le temps de séjour est conservé identique à l'exemple 1, soit 40 minutes. Le volume de liquide réactionnel qui est dépendant de la production et du temps de séjour du catalyseur dans la section réactionnelle est alors identique à celui l'exemple 1. Du fait de la diminution de la surface requise dans les échangeurs, le volume des boucles de recirculation est réduit de 3% (volume de 29,2 m³). Le volume de liquide du réacteur peut alors être augmenté de 8% (volume utile de 12,1m³) ce qui conduit à un gain sur la hauteur de liquide de 8% (Hauteur liquide de 5,4m dans cet exemple). Le diamètre étant indépendant du volume de la boucle de recirculation, il est donc identique à l'exemple 1, à savoir 1,7 m.

La cible de température du mélange 8 de la fraction solvant 7 et de la fraction liquide 4 circulant dans les boucles de recirculation a été conservée identique à l'exemple 1, à savoir 120°C. La température de la fraction liquide refroidie 4, correspondant à la sortie de l'échangeur B, est alors de 121,9°C.

Ainsi, le procédé selon l'invention permet de diminuer la surface d'échange des échangeurs de chaleurs de 13% par rapport à la surface d'échange de l'exemple 1, ce qui représente un gain sur le coût d'opération de l'unité. De plus, la diminution de 3% du volume des boucles de recirculation permet également d'augmenter de 8% le volume de liquide du réacteur, ce qui permet de maximiser la saturation en éthylène dans le liquide contenu dans le réacteur.

La production en héxène-1 est de 9,32 tonnes/heure, la sélectivité en héxène-1 est de 93,2% poids.

### Exemple 3 (selon l'invention)

Le procédé d'oligomérisation selon l'invention est mis en oeuvre dans les mêmes conditions que dans l'exemple 2.

L'étape de refroidissement de la fraction solvant permet de réduire le besoin d'échange dans les boucles de recirculation, qui se traduit par une diminution de la surface des échangeurs. La fraction solvant est refroidie à une température de 40°C. La surface totale des échangeurs de la boucle d'échange est alors limitée à 1440 m² soit une diminution de 13% par rapport à l'exemple 1. Les dimensions du réacteur sont conservées identiques à l'exemple 1. Du fait de la diminution de la surface requise dans les échangeurs, le volume des boucles de recirculation est réduit de 3% (volume de 29,2 m³). Le volume de liquide du réacteur est à 11,2 m³. Le volume de liquide réactionnel de la section réactionnelle est donc alors de 40,4 m³, soit 2% de gain par rapport à l'exemple 1.

La cible de température du mélange 8 du solvant 7 et du fluide de recirculation 4 a été conservée identique à l'exemple 1, à savoir 120°C. La température du flux 4, correspondant à la sortie de l'échangeur B, est alors de 121,9°C.

Le temps de séjour du catalyseur dans la section réactionnelle est alors de 39 minutes.

La production en héxène-1 est de 9,32 tonnes/heure, la sélectivité en héxène-1 est de 93,3% poids.

Le gain sur le temps de séjour permet un gain de sélectivité de 0,1%. Ce gain permet pour une production d'hexene-1 constante, une diminution de la consommation d'éthylène de 0,1% et donc un gain sur le coût d'opération de l'unité.

Le gain sur le temps de séjour permet un gain sur la concentration en chrome nécessaire à la réalisation de ces performances de 2% (correspondant à 0,98 ppm de chrome), soit un gain sur la consommation catalytique et donc un gain sur le coût d'opération de l'unité.

**Le tableau ci-dessous résume les résultats obtenus pour les exemples 1 à 3.**

| | | Exemple 1 | Exemple 2 | Exemple 3 |
|---|---|---|---|---|
| Température de la fraction solvant | °C | 101 | 40 | 40 |
| Température de la fraction liquide refroidie | °C | 120,4 | 121,9 | 121,9 |
| Température fraction solvant + fraction liquide refroidie | °C | 120 | 120 | 120 |
| Surface échange des boucles de recirculation | m² | 1650 | 1440 | 1440 |
| Volume des boucles de recirculation | m³ | 30,1 | 29,2 | 29,2 |
| Volume de liquide du réacteur | m³ | 11,2 | 12,1 | 11,2 |
| Volume section réactionnelle | m³ | 41,3 | 41,3 | 40,4 |
| Diamètre Réacteur | m | 1,7 | 1,7 | 1,7 |
| Hauteur liquide Réacteur | m | 5,0 | 5,4 | 5,0 |
| Temps de séjour | mn | 40 | 40 | 39 |
| Sélectivité | % | 93,2 | 93,2 | 93,3 |

## Revendications

1. Procédé d'oligomérisation d'une charge oléfinique mis en oeuvre à une température comprise entre 30 et 200°C et une pression comprise entre 0,1 et 10 MPa, en présence d'un système catalytique d'oligomérisation homogène et d'un solvant comprenant
a) Une étape d'oligomérisation de ladite charge oléfinique dans une section réactionnelle comprenant un réacteur d'oligomérisation et une ou plusieurs boucles de recirculation permettant le contrôle de la température dans ledit réacteur par le refroidissement d'une fraction de phase liquide,
b) Une étape de séparation dans une section aval de séparation d'un effluent de réaction issu de l'étape a) oligomérisation de façon à obtenir une fraction solvant,
c) Une étape de refroidissement de la fraction solvant issue de l'étape b) à une température inférieure à la température de la ou des boucle(s) de recirculation,
d) Une étape d'introduction à l'étape a) d'oligomérisation de la fraction solvant refroidie issue de l'étape c) dans la section réactionnelle.

2. Procédé selon la revendication 1 dans lequel la fraction solvant issue de l'étape b) est refroidie à une température comprise entre 0 et 150°C.

3. Procédé selon l'une quelconque des revendications précédentes dans lequel la fraction solvant issue de l'étape b) est refroidie à l'étape c) à une température inférieure d'au moins 40°C par rapport à la température de la fraction liquide refroidie dans la boucle de recirculation.

4. Procédé selon l'une quelconque des revendications précédentes dans lequel le refroidissement de la fraction solvant à l'étape c) est réalisé par un ou plusieurs échangeur(s) thermique(s), de préférence choisi(s) parmi un ou plusieurs échangeurs de chaleur du type fluide process/fluide process, de type aéroréfrigérant, de type échangeur à eau de refroidissement.

5. Procédé selon l'une quelconque des revendications précédentes dans lequel la section aval de séparation comprend au moins deux colonnes de distillation, de préférence au moins trois colonnes de distillation, de préférence au moins quatre colonnes de distillation.

6. Procédé selon l'une quelconque des revendications précédentes dans lequel l'étape d) d'introduction de la fraction solvant refroidie est réalisée dans le réacteur et/ou dans une ou plusieurs des boucle(s) de recirculation.

7. Procédé selon l'une quelconque des revendications 4 à 6 dans lequel l'étape d) d'introduction de la fraction refroidie est réalisée dans une boucle de recirculation en amont ou en aval d'un échangeur thermique de ladite boucle de recirculation, de préférence en aval dudit échangeur thermique.

8. Procédé selon l'une quelconque des revendications précédentes dans lequel la fraction solvant refroidie présente un débit en un pourcentage massique par rapport au débit du liquide circulant dans la ou les boucle(s) de recirculation compris entre 0,05 et 15,0%, de préférence entre 0,1 et 10,0%.

9. Procédé selon l'une quelconque des revendications précédentes dans lequel la charge oléfinique comprend des oléfines ayant entre 2 et 6 atomes de carbone, de préférence entre 2 et 4 atomes de carbone.

10. Procédé selon la revendication 9 dans lequel la charge oléfinique est choisie parmi le butène, le propylène, et l'éthylène, seul ou en mélange.

11. Procédé selon l'une quelconque des revendications précédentes dans lequel l'étape a) d'oligomérisation comprend au moins une des sous-étapes suivantes
- sous-étape a1) d'introduction du système catalytique,
- sous-étape a2) de mise en contact avec de la charge oléfinique,
- sous-étape étape a3) de soutirage d'une fraction de phase liquide du réacteur d'oligomérisation,
- sous-étape a4) de refroidissement d'au moins une partie de la fraction liquide soutirée à l'étape a3),
- sous-étape a5) d'introduction dans le réacteur de la fraction liquide refroidie.

12. Procédé selon la revendication 11 dans lequel la sous étape a4) de refroidissement est mise en oeuvre par la circulation d'au moins une partie de la fraction liquide soutirée à la sous-étape a3), au travers d'un ou plusieurs échangeurs thermiques situés dans la boucle de recirculation.

13. Procédé selon la revendication 12 dans lequel le ou les échangeurs thermiques mis en oeuvre à la sous-étape a4) diminue la température de la fraction liquide de 1,0 à 30,0°C, de préférence entre 2,0 et 25,0°C.

14. Procédé selon l'une quelconque des revendications 11 à 13 dans lequel l'effluent de réaction est obtenu par division en deux flux de la fraction liquide soutirée à la sous-étape a3).

15. Procédé selon l'une quelconque des revendications précédentes dans lequel le réacteur d'oligomérisation est choisi parmi un réacteur biphasique gaz/liquide ou monophasique liquide, de manière préférée est un réacteur biphasique gaz/liquide, de manière très préférée de type colonnes à bulles.

## Patentansprüche

1. Verfahren zur Oligomerisation eines olefinischen Einsatzmaterials, das bei einer Temperatur zwischen 30 und 200 °C und einem Druck zwischen 0,1 und 10 MPa in Gegenwart eines homogenen katalytischen Oligomerisationssystems und eines Lösungsmittels durchgeführt wird, umfassend
a) einen Schritt der Oligomerisation des olefinischen Einsatzmaterials in einem Reaktionsabschnitt, umfassend einen Oligomerisationsreaktor und einen oder mehrere Rückführungskreise, die die Regelung der Temperatur in dem Reaktor durch die Kühlung einer flüssigen Phasenfraktion ermöglichen,
b) einen Schritt des Trennens, in einem stromabwärtigen Trennabschnitt, eines aus dem Schritt a) der Oligomerisation hervorgegangenen Reaktionsaustrags, so dass eine Lösungsmittelfraktion erhalten wird,
c) einen Schritt des Kühlens des aus dem Schritt b) hervorgegangenen Lösungsmittelaustrags auf eine Temperatur, die geringer als die Temperatur des oder der Rückführungskreise(s) ist,
d) einen Schritt des Einleitens der aus dem Schritt c) in dem Reaktionsabschnitt hervorgegangenen gekühlten Lösungsmittelfraktion in den Schritt a) der Oligomerisation.

2. Verfahren nach Anspruch 1, wobei die aus dem Schritt b) hervorgegangene Lösungsmittelfraktion auf eine Temperatur zwischen 0 und 150 °C gekühlt wird.

3. Verfahren nach einem der vorhergehenden Ansprüche, wobei die aus dem Schritt b) hervorgegangene Lösungsmittelfraktion im Schritt c) auf eine Temperatur gekühlt wird, die um mindestens 40 °C geringer als die Temperatur der gekühlten flüssigen Fraktion in dem Rückführungskreis ist.

4. Verfahren nach einem der vorhergehenden Ansprüche, wobei das Kühlen der Lösungsmittelfraktion im Schritt c) durch einen oder mehrere Wärmetauscher ausgeführt wird, der(die) vorzugsweise aus einem oder mehreren Wärmetauschern vom Typ Prozessfluid/Prozessfluid, vom Rückkühlertyp, vom Kühlwassertauschertyp ausgewählt ist (sind) .

5. Verfahren nach einem der vorhergehenden Ansprüche, wobei der stromabwärtige Trennabschnitt mindestens zwei Destillationskolonnen, vorzugsweise mindestens drei Destillationskolonnen, vorzugsweise mindestens vier Destillationskolonnen umfasst.

6. Verfahren nach einem der vorhergehenden Ansprüche, wobei der Schritt d) des Einleitens der gekühlten Lösungsmittelfraktion in dem Reaktor und/oder in einem oder mehreren der Rückführungskreis(e) ausgeführt wird.

7. Verfahren nach einem der Ansprüche 4 bis 6, wobei der Schritt d) des Einleitens der gekühlten Fraktion in einem Rückführungskreis stromauf oder stromab eines Wärmetauschers des Rückführungskreises, vorzugsweise stromab des Wärmetauschers ausgeführt wird.

8. Verfahren nach einem der vorhergehenden Ansprüche, wobei die gekühlte Lösungsmittelfraktion einen Volumenstrom in einem Massenprozentsatz bezogen auf den Volumenstrom der in dem oder den Rückführungskreis(en) zirkulierenden Flüssigkeit zwischen 0,05 und 15,0 %, vorzugsweise zwischen 0,1 und 10,0 % aufweist.

9. Verfahren nach einem der vorhergehenden Ansprüche, wobei das olefinische Einsatzmaterial Olefine umfasst, die zwischen 2 und 6 Kohlenstoffatome, vorzugsweise zwischen 2 und 4 Kohlenstoffatome umfassen.

10. Verfahren nach Anspruch 9, wobei das olefinische Einsatzmaterial aus Buten, Propylen und Ethylen, allein oder gemischt, ausgewählt ist.

11. Verfahren nach einem der vorhergehenden Ansprüche, wobei der Schritt a) der Oligomerisation mindestens einen der folgenden Teilschritte umfasst
- Teilschritt a1) des Einleitens des katalytischen Systems,
- Teilschritt a2) des Inkontaktbringens mit olefinischem Einsatzmaterial,
- Teilschritt a3) des Entnehmens einer flüssigen Phasenfraktion aus dem Oligomerisationsreaktor,
- Teilschritt a4) des Kühlens mindestens eines Teils der im Schritt a3) entnommenen flüssigen Fraktion,
- Teilschritt a5) des Einleitens der gekühlten flüssigen Fraktion in den Reaktor.

12. Verfahren nach Anspruch 11, wobei der Teilschritt a4) des Kühlens durch die Zirkulation mindestens eines Teils der im Teilschritt a3) entnommenen flüssigen Fraktion durch einen oder mehrere in dem Rückführungskreis gelegene Wärmetauscher hindurch durchgeführt wird.

13. Verfahren nach Anspruch 12, wobei der oder die im Teilschritt a4) eingesetzten Wärmetauscher die Temperatur der flüssigen Fraktion um 1,0 bis 30,0 °C, vorzugsweise zwischen 2,0 und 25,0 °C verringern.

14. Verfahren nach einem der Ansprüche 11 bis 13, wobei der Reaktionsaustrag durch Teilung in zwei Ströme der im Teilschritt a3) entnommenen flüssigen Fraktion erhalten wird.

15. Verfahren nach einem der vorhergehenden Ansprüche, wobei der Oligomerisationsreaktor aus einem Gas/Flüssigkeits-Zweiphasenreaktor oder einem Flüssigkeits-Einphasenreaktor ausgewählt ist, vorzugsweise ein Gas/Flüssigkeits-Zweiphasenreaktor ist, besonders bevorzugt vom Blasensäulentyp.

## Claims

1. Process for the oligomerization of an olefinic feedstock carried out at a temperature between 30°C and 200°C and a pressure between 0.1 and 10 MPa, in the presence of a homogeneous catalytic oligomerization system and a solvent comprising
a) a step of oligomerizing said olefinic feedstock in a reaction section comprising an oligomerization reactor and one or more recirculation loops enabling the control of the temperature in said reactor via the cooling of a liquid phase fraction,
b) a step of separating, in a downstream separation section, a reaction effluent resulting from the oligomerization step a) so as to obtain a solvent fraction,
c) a step of cooling the solvent fraction resulting from step b) to a temperature below the temperature of the recirculation loop(s),
d) a step of introducing, into the oligomerization step a), the cooled solvent fraction resulting from step c) in the reaction section.

2. Process according to Claim 1, wherein the solvent fraction resulting from step b) is cooled to a temperature between 0°C and 150°C.

3. Process according to either one of the preceding claims, wherein the solvent fraction resulting from step b) is cooled in step c) to a temperature at least 40°C lower relative to the temperature of the cooled liquid fraction in the recirculation loop.

4. Process according to any one of the preceding claims, wherein the cooling of the solvent fraction in step c) is carried out by one or more heat exchangers, preferably chosen from one or more heat exchangers of process fluid/process fluid type, of air cooler type, of cooling water exchanger type.

5. Process according to any one of the preceding claims, wherein the downstream separation section comprises at least two distillation columns, preferably at least three distillation columns, preferably at least four distillation columns.

6. Process according to any one of the preceding claims, wherein step d) of introducing the cooled solvent fraction is carried out in the reactor and/or in one or more of the recirculation loops.

7. Process according to any one of Claims 4 to 6, wherein step d) of introducing the cooled fraction is carried out in a recirculation loop upstream or downstream of a thermal exchanger of said recirculation loop, preferably downstream of said thermal exchanger.

8. Process according to any one of the preceding claims, wherein the cooled solvent fraction has a flow rate, as a weight percentage relative to the flow rate of the liquid circulating in the recirculation loop(s) of between 0.05% and 15.0%, preferably between 0.1% and 10.0%.

9. Process according to any one of the preceding claims, wherein the olefinic feedstock comprises olefins having between 2 and 6 carbon atoms, preferably between 2 and 4 carbon atoms.

10. Process according to Claim 9, wherein the olefinic feedstock is chosen from butene, propylene and ethylene, alone or as a mixture.

11. Process according to any one of the preceding claims, wherein the oligomerization step a) comprises at least one of the following substeps:
- substep a1) of introducing the catalytic system,
- substep a2) of bringing into contact with the olefinic feedstock,
- substep step a3) of withdrawing a liquid phase fraction from the oligomerization reactor,
- substep a4) of cooling at least one portion of the liquid fraction withdrawn in step a3),
- substep a5) of introducing the cooled liquid fraction into the reactor.

12. Process according to Claim 11, wherein the cooling substep a4) is carried out by circulating at least one portion of the liquid fraction withdrawn in substep a3) through one or more thermal exchangers located in the recirculation loop.

13. Process according to Claim 12, wherein the thermal exchanger(s) used in substep a4) reduce(s) the temperature of the liquid fraction by 1.0°C to 30.0°C, preferably between 2.0°C and 25.0°C.

14. Process according to any one of Claims 11 to 13, wherein the reaction effluent is obtained by dividing the liquid fraction withdrawn in substep a3) into two streams.

15. Process according to any one of the preceding claims, wherein the oligomerization reactor is chosen from a two-phase gas/liquid reactor or a single-phase liquid reactor, preferably is a two-phase gas/liquid reactor, very preferably of bubble column type.
